# EUROPÄISCHE PATENTSCHRIFT

(11) **EP 2 789 683 B1**
(45) Veröffentlichungstag und Bekanntmachung des Hinweises auf die Patenterteilung: **23.11.2016**
(21) Anmeldenummer: 13001780.9
(22) Anmeldetag: 08.04.2013
(51) Int. Cl.: C12M 1/00

(54) **Reinigungsgerät**
Cleaning device
Appareil de nettoyage

(43) Veröffentlichungstag der Anmeldung: 15.10.2014
(73) Patentinhaber: Infors AG, 4103 Bottmingen (CH)
(72) Erfinder: Cueni, Dieter, 4222 Zwingen (CH); Egger, Daniel, 4102 Binningen (CH)
(74) Vertreter: Toleti, Martin

(56) Entgegenhaltungen:
- EP-A2- 0 362 863
- WO-A1-98/15362
- US-A- 3 407 121
- US-A1- 2009 130 704
- DATABASE WPI Week 201244 Thomson Scientific, London, GB; AN 2012-H17713 XP002708192, & CN 202 238 816 U (ZHANG Z) 30. Mai 2012 (2012-05-30)
- DATABASE WPI Week 201071 Thomson Scientific, London, GB; AN 2010-M88280 XP002708193, & CN 101 829 505 A (JIANGXI JDL ENVIRONMENTAL PROTECTION RES) 15. September 2010 (2010-09-15)
- DATABASE WPI Week 201233 Thomson Scientific, London, GB; AN 2011-Q41456 XP002708194, & CN 102 258 944 A (UNIV TONGJI) 30. November 2011 (2011-11-30)

## Beschreibung

Die Erfindung betrifft ein gattungsgemässes Reinigungsgerät für einen Laborfermenter nach dem Oberbegriff des Hauptanspruchs, nämlich ein solches für einen Laborfermenter zum Züchten von Mikroorganismen wie Bakterien- oder Pilzkulturen oder Zellkulturen, wobei der Laborfermenter mit einem Sparger genannten Gaszuführanschluss, der an eine bis in die Nährlösung reichende Leitung angeschlossen ist, mit einem zu dem über der Nährlösung befindlichen Gasraum führenden, ggf. mit einem Abgaskühler auf der Aussenseite des Laborfermenters verbundenen Abgasanschluss und mit einem Entnahmeanschluss für die Nährlösung oder Reinigungsflüssigkeit versehen ist, der bis zu der tiefsten Stelle in dem Fermenter führt. Daneben kann der Laborfermenter die an sich bekannten weiteren Bestandteile wie Rührwerk, Messsensoren, Heizung sowie die Zuführung von Lauge, Säure, AF, Feed etc über Pumpen und push-Ventile sowie Luftzuführungen von kleiner 1, vorzugsweise 0,5 bar aufweisen.

Ein solches Reinigungsgerät für Teile von Laborfermentern bis ca. 30 Liter Fassunngsvermögen ist als Autoklav an sich bekannt.

Um diesen Laborfermenter nach seinem Einsatz reinigen zu können, ist es erforderlich, ihn zu zerlegen und die kleineren Teile zunächst zu reinigen sind danach in einen Autoklav einzulegen und diese danach zu autoklavieren. Nach der dabei erfolgten Sterilisation müssen die Teile wieder zu dem Laborfermenter steril zusammengesetzt werden, damit dieser wieder einsatzfähig ist. Für diese bekannte Art der Reinigung wird mehr als ein ganzer Tag benötigt, die überdiese von einem Chemielaborant erbracht werden muss. Damit ist das Reinigen von Laborfermentern sehr aufwendig.

Aus der US 2009/0130704 ist ein Bioreaktor bekannt, der zu Reinigungszwecken einen in den Deckel des Reaktors integrierten Sprühball aufweist.

Aus der EP 0 362 863 A2 ist eine Säuberungs- und Befüllanlage für Kapseln bekannt, die an Säuberungs- und Befüllstationen der aseptischen Anlage vorbeigeführt werden.

Der Erfindung liegt daher die Aufgabe zugrunde, ein weniger aufwendig arbeitendes Reinigungsgerät für einen Laborfermenter bereitzustellen.

Diese Aufgabe wird bei einem gattungsgemässen Reinigungsgerät für einen Laborfermenter nach dem Oberbegriff des Hauptanspruchs erfindungsgemäss durch dessen kennzeichnende Merkmale, also dadurch gelöst, dass das Reinigungsgerät drei Reinigungsanschlüsse, nämlich einen Tauchrohranschluss sowie zwei Gasanschlüsse aufweist, dass jeder Gasanschluss an ein einen Ausgang aufweisendes Zwischenschaltventil geführt ist, dass jedes Zwischenschaltventil zwei Schaltstellungen, nämlich eine den Gasanschluss zu dem Ausgang durchlassenden Reinigungsstartsowie eine diese Verbindung schliessende Reinigungsendstellung aufweist, dass die Reinigungsanschlüsse über Leitungen mit Steuerventilen, einer Pumpe, einem Filter sowie vermittels einer einen Reinigungsvorgang steuernden Reinigungssteuerung mit einem Luft-, einem Wasser- sowie einem Abwasseranschluss verbunden sind, und dass die Leitungen als Schlauchleitungen, die Ventile als Schlauchquetschventile und die Pumpe als Schlauchquetschpumpe ausgebildet sind.

Das erfindungsgemäss transportable Reinigungsgerät wird mit seinem Tauchrohranschluss an den Entnahmeanschluss und mit seinen beiden Gasanschlüssen über die sich in der Reinigungsstartstellung befindenden Zwischenventile an den Gaszuführanschluss und den Abgasanschluss des zu reinigenden Laborfermenters angeschlossen. Nach Zugabe von Lauge, z.B. Natronlauge und/oder Wasser in den Laborfermenter zum Zwecke dessen Reinigung wird bei dem Reinigungsvorgang das Gemisch ggf. unter Umkehr seiner Fliessrichtung und ggf. Zugabe von Wasser von dem Wasseranschluss ausreichend lange zirkuliert, danach durch Zugabe von Säure, z.B. Phosphorsäure neutralisiert und schliesslich durch Zugabe von Luft in den Gasraum über den Entnahmeanschluss des Laborfermenters zu dem Tauchrohranschluss und letztlich zu dem Abwasseranschluss transportiert, der an einen sogenannten Killtank angeschlossen werden kann. Nach dem Ende des Reinigungsvorganges können alle Schlauchanschlüsse zu dem Laborfermenter an den Reinigungsanschlüssen unter Belassung der Zwischenventile noch in deren Reinigungsstartstellung an dem Laborfermenter getrennt und ggf. verschlossen werden, der dann für ein neuen Fementierzyklus vollständig gereinigt bereit steht, ohne dass es seiner Zerlegung in Teile sowie der Autoklavierung derselben bedarf. Das erfindungsgemässe Reinigungsgerät kann auch autark mit einem Wasser-und einem auch Killtank genannten Abwassertank sowie eine elektrische Batterie für die Pumpe sowie die Reinigungssteuerung versehen sein. Es ist natürlich auch möglich, dass statt dessen externe Tanks und das elektrische Wechselstromnetz verwendet werden.

Damit die Reinigungsarbeit nicht von dem Laborfermenter auf das erfindungsgemässe Reinigungsgerät verlagert wird, werden von diesem sämtliche, und damit alle miteinander zusammenhängenden Schlauchleitungen nach Art eines Schlauchleitungsbaumes als Schlauchleitungsatz von den Schlauchquetschventilen und der Schlauchquetschpumpe nach einigen Einsätzen gelöst und gegen einen sauberen neuen, nicht notwendigerweise sterilen Schlauchleitungsatz ausgetauscht, weil dieser von der zunächst durchgespülten Lauge gereinigt und sterilisiert wird, womit das Reinigungsgerät auch wieder für einen nächsten Reinigungsvorgang einsatzbereit ist.

Weitere zweckmässige Ausgestaltungen und Weiterbildungen der Erfindung sind in den Unteransprüchen gekennzeichnet.

Ein Ausführungsbeispiel der Erfindung wird nachfolgend unter Bezugnahme auf die Zeichnung erläutert. In dieser zeigt:
Figur 1 : ein Reinigungsgerät sowie einen Laborfermenter mit Steuereinheit, in perspektivischer Darstellung;
Figur 2 : das Reinigungsgerät gemäss Figur 1 und
Figur 3 : ein Schaltschema für das Reinigungsgerät und den Laborfermenter.

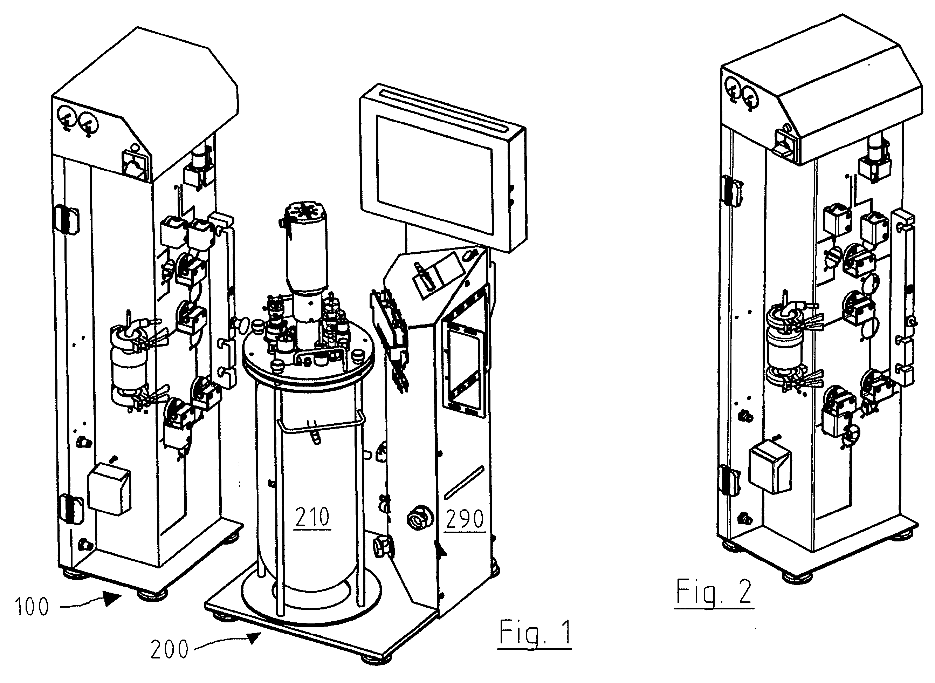

Das Reinigungsgerät 100 für einen Fermenter 200 zum Züchten von Bakterien- oder Pilzkulturen, ist in schematischer Darstellung in Figur 1 dargestellt. Der Fermenter 200 weist eine Reinigungssteuerung 290 und den eigentlichen Laborfermenter 210 auf. Dieser kann vorzugsweise an seinem Deckel mit an sich bekannten weiteren Bestandteilen wie Rührwerk nebst Motor M, Messsensoren, Heizung sowie die Zuführung von Lauge, Säure, AF, Feed etc. über Pumpen P und push-Ventile sowie Luftzuführungen von kleiner 1, vorzugsweise 0,5 bar versehen sein, die in diesem Zusammenhang nicht näher interessieren und deshalb nicht näher beschrieben werden. Ausserdem ist über ein CIP/SIP-push-Ventil dem Laborfermenter 210 als Base eine Natronlauge von einer in einem Teil 190 des Reinigungsgerätes 100 befindliche Pumpe P3 sowie als Säure Phosphorsäure von einer Pumpe P2 zum Zwecke der Reinigung bzw. nach dieser zur Neutralisation zuführbar.

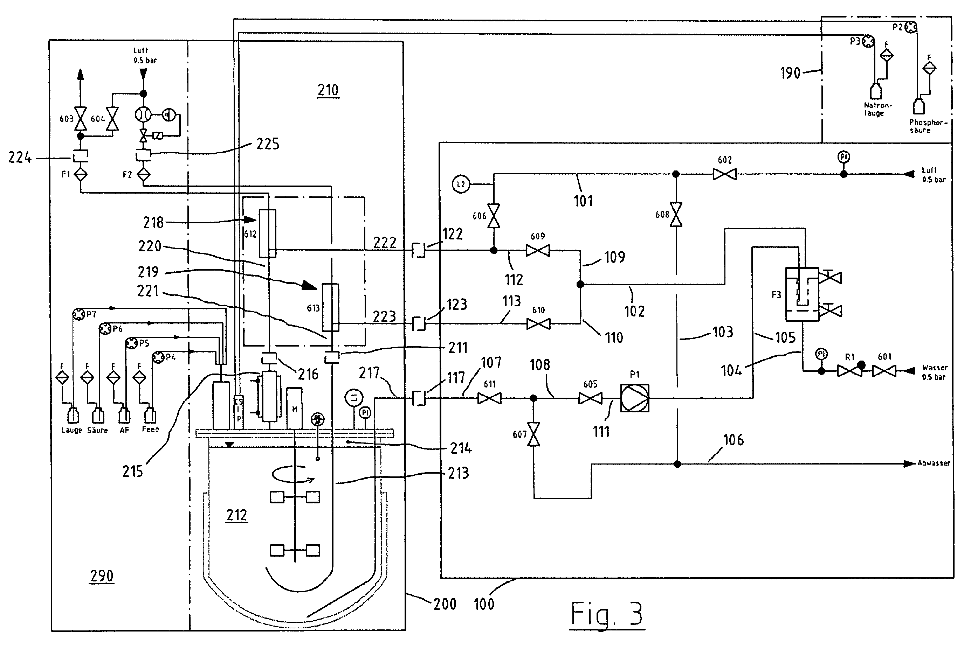

Ausserdem weist der Laborfermenter 210, wie Figur 3 zeigt, einen "Sparger" genannten Gaszuführanschluss 211, der an eine bis in die Nährlösung 212 reichende Leitung 213 angeschlossen ist, einen zu dem über der Nährlösung 212 befindlichen Gasraum 214 führenden, ggf. mit einem Abgaskühler 215 auf der Aussenseite des Laborfermenters 210 verbundenen Abgasanschluss 216 und einem Entnahmeanschluss 217 für die Nährlösung auf, der bis zu der tiefsten Stelle in dem Laborfermenter 210 führt. Der Gaszuführanschluss 211 und der Abgasanschluss 216 sind an je ein, einen Ausgang 220,221 aufweisendes Zwischenschaltventil 218,219 mit zwei Schaltstellungen geführt, das als 3/2-Ventil ausgebildet ist und dessen Ausgang in der einen Schaltstellung, nämlich der Reinigungsstartstellung entweder mit Reinigungsanschlüssen 222,223 oder in der anderen Schaltstellung, nämlich der Reinigungsendstellung oder Betriebsstellung des Laborfermenters nur mit Anschlüssen 224,225 der Reinigungssteuerung 290 im Betrieb verbunden.

Das Reinigungsgerät 100 ist mit Ventilen 601,602 sowie 605 bis 610, einer Umwälzpumpe P1, einem Filter F3 sowie vermittels einer einen Reinigungsvorgang steuernden -nicht dargestellten- Reinigungssteuerung mit einem Luft-, einem Wasser- sowie einem Abwasseranschluss verbunden, welche Reinigungssteuerung nach Zugabe von Natronlauge und/oder Wasser von dem Teil 190 in den Laborfermenter 210 zum Zwecke dessen Reinigung des während des Reinigungsvorgangs das Gemisch ggf. unter Umkehr seiner Fliessrichtung ausreichend lange zirkuliert, danach durch Zugabe von Phosphorsäure als Säure neutralisiert und schliesslich durch Zugabe von Luft in den Gasraum 214 über den Entnahmeanschluss 217 des Laborfermenters zu einem Tauchrohranschluss 117 des Reinigungsgerätes 100 und letztlich zu dem Abwasseranschluss transportiert, der an einen sogenannten Killtank angeschlossen werden kann.

Zum temporären Anschluss an den Laborfermenter 210 zum Zwecke dessen Reinigung weist das Reinigungsgerät 100 drei Reinigungsanschlüsse 117,122,123, nämlich einen Tauchrohranschluss 117 sowie zwei Gasanschlüsse 122,123 auf, welch beiden letztere an das den Ausgang 220,221 aufweisende Zwischenschaltventil 218,219 geführt sind. Nach Durchführung der Reinigung wird das Reinigungsgerät 100 an seinen drei Reinigungsanschlüssen 117,122,123 von dem Laborfermenter 210, an dem die Zwischenventile 218,219 verbleiben, getrennt und erforderlichenfalls mit Stopfen verschlossen. Der Laborfermenter 210 ist dann für einen neuen Einsatz bereit.

Schliesslich sind alle Leitungen 101-113 in dem Reinigungsgerät 100 als zusammenhängende, miteinander verbundene Schlauchleitungen, die Ventile 601,602 sowie 605 bis 610 als Schlauchquetschventile und die Umwälzpumpe P1 als Schlauchquetschpumpe ausgebildet. Hierdurch bedingt wird die Reinigungsarbeit nicht von dem Laborfermenter 210 auf das erfindungsgemässe Reinigungsgerät 100 verlagert, denn es können dessen sämtliche, und damit alle miteinander zusammenhängenden Schlauchleitungen nach Art eines Schlauchleitungsbaumes als Schlauchleitungsatz von den Schlauchquetschventilen und der Schlauchquetschpumpe nach einigen Einsätzen gelöst und gegen einen sauberen neuen Schlauchleitungsatz ausgetauscht, womit auch das Reinigungsgerät wieder für den nächsten Reinigungsvorgang einsatzbereit ist.

## Patentansprüche

1. Reinigungsgerät (100) für einen Laborfermenter (210) zum Züchten von Mikroorganismen wie Bakterien- oder Pilzkulturen oder Zellkulturen, wobei der Laborfermenter (210) mit einem Sparger genannten Gaszuführanschluss (211), der an eine bis in die Nährlösung (212) reichende Leitung (213) angeschlossen ist, mit einem zu dem über der Nährlösung befindlichen Gasraum (214) führenden, ggf. mit einem Abgaskühler (215) auf der Aussenseite des Laborfermenters (210) verbundenen Abgasanschluss (216) und mit einem Entnahmeanschluss (217) für die Nährlösung versehen ist, der bis zu der tiefsten Stelle in dem Laborfermenter führt, **dadurch gekennzeichnet,**
- **dass** das Reinigungsgerät (100) transportabel ist,
- **dass** das Reinigungsgerät (100) drei Reinigungsanschlüsse (117,122,123), nämlich einen Tauchrohranschluss (117) sowie zwei Gasanschlüsse (122,123) aufweist,
- **dass** das Reinigungsgerät (100) ausgestaltet ist zum temporären Anschluss an den Laborfermenter (210) zum Zwecke dessen Reinigung, durch Führen eines jeden Gasanschlusses (122,123) über ein einen Ausgang (220,221) aufweisendes Zwischenschaltventil (218,219) an den Gaszuführanschluss (211) und den Abgasanschluss (216) des Laborfermenters (210), wobei
jedes Zwischenschaltventil (218,219) zwei Schaltstellungen, nämlich eine den Gasanschluss (122,123) zu dem Ausgang (220,221) durchlassenden Reinigungsstart- sowie eine diese Verbindung schliessende Reinigungsendstellung aufweist, und durch Anschliessen des Tauchrohranschlusses (117) an den Entnahmeanschluss (211) des Laborfermenters (219),
- **dass** die Reinigungsanschlüsse (117,122,123) über Leitungen (101-113) mit Ventilen (601,602 sowie 605-610), einer Umwälzpumpe (P1), zumindest einem Filter (F3) sowie vermittels einer den Reinigungsvorgang steuernden Reinigungssteuerung mit einem Luft-, einem Wasser- sowie einem Abwasseranschluss verbunden sind,
- und **dass** die Leitungen (101-113) als Schlauchleitungen, die Ventile als Schlauchquetschventile und die Umwälzpumpe (P1) als Schlauchquetschpumpe ausgebildet sind.

2. Reinigungsgerät nach Anspruch 1, **dadurch gekennzeichnet, dass** es auch einen Teil (190) mit einer Lauge, z.B. Natronlauge liefernde Pumpe (P3) sowie Säure, z.B. Phosphorsäure liefernden Pumpe (P2) aufweist.

## Claims

1. Cleaning device (100) for a laboratory fermenter (210) for growing microorganisms like bacteria cultures or fungal cultures or cell cultures, wherein the laboratory fermenter (210) has a gas supply port (211) called sparger, which is connected to a pipe (213) extending into the nutrient solution (212), with an exhaust port (216) guiding to the gas space (214) located above the nutrient solution, which is connected to an exhaust cooler (215) on the outside of the laboratory fermenter (210) if necessary, and with an extraction port (217) for the nutrient solution, which extends up to the deepest point in the laboratory fermenter, **characterized in that**
- the cleaning device (100) is transportable,
- the cleaning device (100) has three cleaning ports (117, 122, 123), namely an immersion pipe port (117) as well as two gas port (122, 123),
- the cleaning device (100) is adapted to temporarily connect with the laboratory fermenter (210), with the aim of cleaning it, by guiding each gas port (122, 123) to the gas supply port (211) and the exhaust port (216) of the laboratory fermenter (210) via an intermediary switching valve (218, 219) having an outlet (220, 221), wherein
each intermediary switching valve (218, 219) has two switching positions, that is: a cleaning start position opening a connection between the gas port (122, 123) and the outlet (220, 221) as well as a cleaning end position closing this connection, and by connecting the immersion pipe port (117) to the extraction port (211) of the laboratory fermenter (219),
- the cleaning ports (117, 122, 123) are connected via pipes (101-113) with valves (601, 602 as well as 605-610), with a circulation pump (P1), at least a filter (F3) as well as with an air port, with a water port as well as a waste water port by means of a cleaning controller controlling the cleaning process,
- and the pipes (101-113) are formed as hose pipes, the valves as constriction hose valves and the circulation pump (P1) as peristaltic pump.

2. Cleaning device according to claim 1, **characterized in that** it also has a part (190) with a pump (P3) delivering lye, e.g. caustic soda, as well as with a pump (P3) delivering acid, e.g. phosphoric acid.

## Revendications

1. Dispositif de nettoyage (100) pour un fermenteur de laboratoire (210) pour cultiver des microorganismes comme des cultures bactériales ou des cultures fongiques ou des cultures de cellules, le fermenteur de laboratoire (210) ayant un connecteur d'alimentation de gaz (211) dit sparger, qui est connecté avec un conduit (213) qui s'étend dans la solution nutritive (212), avec un connecteur d'échappement (216) qui mène dans un espace de gaz (214) situé au-dessus de la solution nutritive, qui est connecté avec un refroidisseur d'échappement (215) à l'extérieur du fermenteur de laboratoire (210) si nécessaire, et avec un connecteur d'extraction (217) pour la solution nutritive, qui s'étend jusqu'à le point le plus profond dans le fermenteur de laboratoire, **caractérisé en ce que**
- le dispositif de nettoyage (100) est transportable,
- le dispositif de nettoyage (100) a trois connecteurs de nettoyage (117, 122, 123), c'est-à-dire un connecteur de tube plongeur (117) ainsi que deux connecteurs de gaz (122, 123),
- le dispositif de nettoyage (100) est adapté à se connecter temporairement avec le fermenteur de laboratoire (210), avec le but de le nettoyer, en guidant chaque connecteur de gaz (122, 123) au connecteur d'alimentation de gaz (211) et au connecteur d'échappement (216) du fermenteur de laboratoire (210) par l'intermédiaire d'une soupape de commutation intermédiaire (218, 219) ayant une sortie (220, 221)
chaque soupape de commutation intermédiaire (218, 219) ayant deux position de commutation, c'est-à-dire une position de démarrage du nettoyage, qui ouvre une connexion entre le connecteur de gaz (122, 123) et la sortie (220, 221), ainsi qu'une position de fin du nettoyage qui ferme cette connexion, et en connectant le connecteur de tube plongeur (117) avec le connecteur d'extraction (211) du fermenteur de laboratoire (219),
- les connecteurs de nettoyage (117, 122, 123) sont connectés avec des soupapes (601, 602 ainsi que 605-610) par des conduits (101-113), avec une pompe de circulation (P1), au moins un filtre (F3) ainsi qu'avec un connecteur d'air, un connecteur d'eau ainsi qu'un connecteur d'eau de décharge à l'aide d'une commande de nettoyage qui commande le processus de nettoyage,
- et les conduits (101-113) ont une forme de tuyau, les soupapes ont une forme de soupape péristaltique et la pompe de circulation (P1) ont une forme de pompe péristaltique.

2. Dispositif de nettoyage selon la revendication 1, **caractérisé en ce qu'**il a en outre une composante (190) avec une pompe (P3) qui délivre de la lessive, par exemple de la soude caustique, ainsi qu'une pompe (P3) qui délivre d'acide, par exemple d'acide phosphorique.
